# EUROPEAN PATENT SPECIFICATION

(11) **EP 4 314 369 B1**
(45) Date of publication and mention of the grant of the patent: **30.07.2025**
(21) Application number: 22709615.3
(22) Date of filing: 08.02.2022
(51) Int. Cl.: C22C 18/00, C22C 23/00, B21C 23/08, C22C 23/02, B21C 23/00, B21C 29/00, B21C 23/21, A61F 2/82, A61L 31/02, A61L 31/14, A61F 2/24

(54) **METHOD AND DEVICE FOR PRODUCING A TUBULAR SEMI-FINISHED PRODUCT FOR A SCAFFOLD OF AN IMPLANT**
VERFAHREN UND VORRICHTUNG ZUR HERSTELLUNG EINES RÖHRENFÖRMIGEN HALBFERTIGPRODUKTS FÜR EIN GERÜST EINES IMPLANTATS
PROCÉDÉ ET DISPOSITIF DE FABRICATION D'UN DEMI-PRODUIT TUBULAIRE POUR LE SQUELETTE D'UN IMPLANT

(30) Priority: 24.03.2021 EP 21164643
(43) Date of publication of application: 07.02.2024
(73) Proprietor: BIOTRONIK AG, 8180 Bülach (CH)
(72) Inventor: HEUSS, Alexander, 23999 Insel Poel (DE); BAYER, Ullrich, 18209 Bad Doberan (DE); ANOPUO, Okechukwu, 18109 Rostock (DE); SCHOOF, André, 18209 Bad Doberan (DE); BLOCK, Bernd, 71229 Leonberg (DE); HANNEMANN, Jan, 18507 Rostock (DE)
(74) Representative: Biotronik Corporate Services SE
(86) International application number: PCT/EP2022/052951
(87) International publication number: WO 2022/199922

(56) References cited:
- EP-A1- 1 835 042
- WO-A1-2017/204803
- GOLOVKO A: "Manufacture of small diameter magnesium tubes with the hot extrusion and drawing processes", TUBE UKRAINE 2007 INTERNATIONAL CONFERENCE, 24-26 SEPTEMBER 2007, DNEPROPETROVSK, UKRAINE,, 24 September 2007 (2007-09-24), pages 263 - 274, XP009182084, ISBN: 978-1-60423-867-9
- SILVA CLÁUDIO L ET AL: "Effect of severe plastic deformation on the biocompatibility and corrosion rate of pure magnesium", JOURNAL OF MATERIAL SCIENCE, KLUWER ACADEMIC PUBLISHERS, DORDRECHT, vol. 52, no. 10, 31 January 2017 (2017-01-31), pages 5992 - 6003, XP036168619, ISSN: 0022-2461, [retrieved on 20170131], DOI: 10.1007/S10853-017-0835-X
- CHEN JUNXIU ET AL: "Mechanical properties of magnesium alloys for medical application: A review", JOURNAL OF THE MECHANICAL BEHAVIOR OF BIOMEDICAL MATERIALS, ELSEVIER, AMSTERDAM, NL, vol. 87, 21 July 2018 (2018-07-21), pages 68 - 79, XP085469459, ISSN: 1751-6161, DOI: 10.1016/J.JMBBM.2018.07.022

## Description

The invention relates to a method for producing a tubular semi-finished product for a structural scaffold of a medical implant, wherein the semi-finished product consists of a magnesium alloy and/or a zinc alloy, and to a corresponding device and a semi-finished product produced by means of the method. The structural scaffold will be referred to hereinafter as a scaffold. The invention also relates to an implant having a structural scaffold in the form of a tubular basic structure.

Bioresorbable scaffolds formed from a magnesium alloy are used in resorbable implants intended for vascular surgery, for example coronary or peripheral implants, for example for stents or in heart valve replacement. The scaffolds are produced by being cut out from a tubular semi-finished product, for example by means of laser. The scaffolds are often introduced minimally invasively into the body of the patient. For implantation of this kind, the scaffold has a state with a small diameter, in order to transport it, for example along the blood vessels of the patient, to the site of the treatment. There, such a scaffold is then transferred, for example by means of a balloon, into a state with a larger diameter (expanded, dilated) in order to perform the desired function in the body of the patient, for example a supporting function for a vessel.

Bioresorbable scaffolds formed from magnesium alloys, on account of their hexagonal lattice structure and their limited number of glide planes, have fundamental disadvantages in plastic forming processes, such as dilation. In the past it has therefore been attempted to provide improvements in respect of tubular semi-finished products for the scaffold by way of alloying and process-related measures. In that case, tests leading to an optimised grain size and the most homogeneous distribution possible of intermetallic phases played a key role. This was usually achieved - with a predefined alloy composition - by the optimisation of thermomechanical forming processes.

Semi-finished tubes can be produced in different ways. Known methods, such as tube drawing, rod drawing or also extrusion bring about a preferred orientation of the grains in the microstructure. This anisotropic microstructure or grain structure significantly influences the mechanical properties of the implant scaffolds produced from the semi-finished products. The semi-finished tubes have advantageous mechanical properties in the direction of their longitudinal axis. This circumstance, however, which is favourable for the tube properties, cannot be utilised to the fullest extent in a scaffold manufactured from these tubes, since this scaffold must also withstand stress moments during the dilation process which do not coincide with the deformation direction.

For example, in the known multi-stage tube drawing process with interstage annealing, the limited formability at room temperature caused by the hexagonal, very close-packed magnesium lattice is taken into consideration. For this reason, only small degrees of forming can be achieved per drawing step at room temperature. The deformation capability is improved by means of intermediate annealing steps, and the semi-finished product in the form of an internally bored blank is deformed until the desired end dimension is achieved. The recurrent deformation capability requires a fully recrystallised microstructure. If this is not achieved after intermediate annealing steps, destruction of the semi-finished product or irreversible crack formation cannot be ruled out. Due to the numerous forming steps and the necessary intermediate annealing steps, this method is very disadvantageous from an economical viewpoint as well.

A single-stage warm extrusion by means of forward or backward hollow extrusion is also prior art. In such an extrusion process, a bored blank is pressed through a heated die. The thermal influences acting during this process are decisive for the characteristics of the mechanical properties. A high forming temperature causes a complete recrystallisation in the microstructure and thus approximately isotropic material properties. However, in the known method, grain growth occurs, which can reduce the tensile strength and elongation at break. In this regard, an extrusion method along with an extrusion tool was disclosed by Golovko at the Tube Ukraine 2007 international conference, on September 24-26^{th} 2007 in Dnepropetrovsk, summarized in the conference magazine at pages 263-274. Alternatively, with a low forming temperature the grain growth is prevented or hindered. Due to the low temperature, however, the microstructure is only partly dynamically recrystallised, resulting in a strong texture, which results in highly anisotropic material properties.

The objective of the present invention is therefore to minimise or overcome the above-mentioned disadvantages. A method is described and a device created which improve the dilatability of an implant scaffold produced from the semi-finished product.

The above object is achieved by the method described in claim 1 and by the device described in claim 4.

In particular, the method according to the invention has the following steps:
a) extruding the tubular semi-finished product by means of a heated die or tempering the extruded tubular semi-finished product by means of a heating device,
b) introducing into the material of the semi-finished product exiting from the die or the heating device a tensile stress and/or a torsional stress by means of a tube drawing device, which has a clamping device, wherein the clamping device is fixed on a predefined portion of the tubular semi-finished product and the tensile force generated by the tube drawing device and/or the torsion moment generated by the tube drawing device transfers to the semi-finished product.

In one exemplary embodiment, the die for extruding the tubular semi-finished product is part of an extrusion device which, besides the die, also comprises a ram.

Accordingly, a device according to the invention (tube drawing device) comprises a clamping device, wherein the device is designed in such a way that it generates a tensile stress and/or a torsional stress in the material of the semi-finished product, wherein the tensile force generated for this purpose by the device and/or the torsion moment generated by the tube drawing device is transferable by means of the clamping device to the material of the semi-finished product exiting from the die or the heating device, wherein the clamping device is fixable on a predefined portion of the tubular semi-finished product.

The material of the tubular semi-finished product comprises a magnesium alloy, for example WE43, magnesium-zinc-aluminium, magnesium-aluminium, or magnesium-zinc-calcium. Here, preferably ultra-pure magnesium alloys are used, such as magnesium-zinc-aluminium with 0-4 % by weight Zn and 2-10 % by weight Al or with 1.5-7% by weight Zn and 0.5-3.5 % by weight Al, such as magnesium-aluminium with 5-10 % by weight Al, in particular 5.5-7 % by weight, particularly preferably 6.25% aluminium, such as magnesium-zinc-calcium with 3-7 % by weight Zn and 0.001-0.5 % by weight Ca or 0-3 % by weight Zn and 0-0.6 % by weight Ca. Such ultra-pure magnesium alloys expediently contain, besides the stated alloy elements, less than 0.006 % by weight of other elements (impurities such as Fe, Cu, Co, Si etc. or rare earths).

Alternatively or in combination, the material of the tubular semi-finished product comprises a zinc alloy, in particular a zinc-magnesium-calcium alloy with 0.2 to 3 % by weight Mg, in particular 0.5 to 1.5 % by weight Mg, and 0 to 1.5 % by weight Ca, in particular 0.01 to 0.5 % by weight Ca, wherein the remainder is formed by zinc and unavoidable impurities.

The fundamental concept of the inventors that is implemented by the method according to the invention and the device according to the invention is based on the introduction of additional stresses (tensile and/or torsional stresses) into the tubular semi-finished product. This can be introduced into the tube directly after the tube extrusion process (performed for example at elevated temperatures) or later within the scope of an additional thermally assisted finishing step by means of the heating device. A metallographic microstructure thus results, which contains grains, of which the preferred orientation does not correspond to the direction of the tube axis and thus the deformation direction of the semi-finished product. The preferred orientation of many grains of the microstructure produced by means of the method according to the invention runs obliquely or transversely to the longitudinal axis of the tubular semi-finished product, so that a mixed grain orientation is created on the whole. The advantage of this mixed grain orientation then takes effect if scaffolds which are fabricated from these tubes are mechanically loaded and plastically deformed during the dilation process. The scaffold regions that have the greatest plastic expansion during the dilation process are identical to the points of origin of cracks which lead to a later failure of the scaffold. On account of the mixed grain orientation provided with natural torsional stresses, the crack propagation that occurs with ongoing plastic deformation of the scaffold is slowed, so that a scaffold produced from the semi-finished product produced in accordance with the invention fails later on average. Consequently, the dilatability or the plastic deformation capability of an implant scaffold produced from the semi-finished product is improved and larger dilation diameters can be used which are extremely desirable from the viewpoint of clinical safety and in addition significantly increase the safety of this class of medicinal product.

The method according to the invention, which is performed with the device according to the invention, additionally causes a grain refinement. This grain refinement, besides the formation of new grains, also comprises the above-described effect in relation to the grain orientation, which in the event of a mechanical load of the scaffold produced from the semi-finished product, advantageously counteracts crack propagation. The solution according to the invention is tailored to the situation of plastic deformation, which is accompanied by crack propagation, wherein the grains of the semi-finished product according to the invention (and thus also of the scaffold produced therefrom) delay crack propagation on account of their orientation. Due to the applied tensile and/or torsional stress, a recrystallisation of amorphous regions of the microstructure is brought about, wherein the grain orientation of the growing grains is different and in particular runs obliquely or transversely to the longitudinal axis of the tubular semi-finished product. This state is "frozen" in each grain once the recrystallisation is not reached.

A suitable temperature range for the material of the semi-finished product heated in the die and consisting of a magnesium alloy is between 240°C and 290°C.

A suitable temperature range for the material of the semi-finished product heated in the die and consisting of a zinc alloy is between 140°C and 310°C, in particular between 150°C and 250°C. Alternatively, a suitable temperature range for the tempering of an extruded tubular semi-finished product consisting of a magnesium alloy for preparation for the effect of the tensile and/or torsional stress is between 250°C and 450°C.

For generation of the tensile force, the tube drawing device has a slide (puller) which is movable on or in a guide and which has a clamping device fastened thereto, wherein the guide of the slide is arranged on a corresponding holding device or a corresponding stand. The guide can be formed, for example, as a dovetail guide. To move the slide in the guide, a stepper motor or a pneumatic drive is provided, for example. The movement of the slide generates the tensile force, which is transferred to the material of the semi-finished product via the clamping device connected to the slide. If the slide is driven pneumatically, for example, a force/distance diagram can be specified and implemented by the use of electronic proportional valves.

The clamping device can additionally be mounted rotatably and likewise provided with a a pneumatic drive, wherein the longitudinal axis of the rotation runs parallel to the longitudinal axis of the tubular semi-finished produced held by means of the clamping device. The clamping device transfers a torsion moment to the semi-finished product, wherein the torsional stress can be adjusted as a function of the tube length.

The clamping device in the starting state is preferably arranged a few tenths of a millimetre to 5 mm above the die and engages the semi-finished product with parallel grippers or also with a centric gripper. The distance of the gripper from the die increases over the tube length. The clamping diameter of the grippers can be adjusted, for example by means of a millimetre screw.

The above object is also achieved by a system comprising an above-mentioned device (tube drawing device) and
- an extrusion device with a heated die for extruding the tubular semi-finished product or
- a heating device for tempering an extruded tubular semi-finished product.

According to the invention has the device a slide (17) which is movable on or in a guide, which generates the tensile force, and which is connected to the clamping device (19) and the slide (17) is driven pneumatically The temperature ranges for the die of the extrusion device or the heating device that are advantageous for a magnesium alloy or zinc alloy have already been stated above.

The above object is also achieved correspondingly by a tubular semi-finished product for an implant scaffold produced by means of the above-mentioned method and by an implant scaffold produced by cutting from the tubular semi-finished product, for example by means of laser cutting. The cut-out scaffold can then be electropolished and coated as necessary.

The above object is likewise achieved by an implant for implantation in a bodily lumen, wherein the implant comprises a tubular basic structure which contains a magnesium alloy and/or zinc alloy, wherein the magnesium and/or zinc alloy has a grain structure formed from uniform and equally distributed grains with a mixed grain orientation, wherein the tubular basic structure in a starting state has a plurality of bars which are oriented at least in part in the circumferential direction. The implant can be compressed by a plastic deformation and expanded by a later plastic deformation to up to 150% of the diameter in the starting state, without any of the bars oriented at least in part in the circumferential direction breaking.

A bar oriented at least in part in the circumferential direction is understood within the scope of the application to mean a bar that has an angle of <90°, in particular <50° with the circumferential direction. A bar that has an angle of 0° with the circumferential direction is oriented accordingly in the circumferential direction. A bar, often also referred to as a strut, is understood to mean an elongate structure that is straight or wound, in particular wound in an S shape. The orientation of a structure wound in this way corresponds to the centre of gravity vector.

An implant for implantation in a bodily lumen is in particular a stent for implantation in a blood vessel.

The starting state is understood to mean the implant as it is obtained after having been cut out from the tubular semi-finished product and after optional electropolishing and/or coating.

The starting state of the implant is thus the state before the implant is compressed onto a catheter, in particular a balloon catheter, for insertion of the implant. The compression and thus fastening of the implant on the catheter is often also referred to as crimping.

A breakage of the bars is understood within the scope of the application to mean a crack that passes at least once through the entire cross section of the bars.

The grains in one embodiment have a mean grain size of at most 15 micrometres, preferably at most 10 micrometres.

The invention will be explained hereinafter on the basis of an exemplary embodiment and with reference to the figures. All features that are described and/or shown in the figures, individually or in any combination, form the subject matter of the invention, also independently of their compilation in the claims or the dependency references of the claims.

The figures show schematically:
- Fig. 1: a system according to the invention with a device according to the invention in a view from the side, partly in section,
- Fig. 2: a microsection of the microstructure of an extruded tubular semi-finished product formed from a magnesium alloy of magnesium and 6.25 % by weight aluminium, which was extruded at 275°C without the introduction according to the invention of a tensile stress and/or torsional stress,
- Fig. 3: a microsection of the microstructure of an extruded tubular semi-finished product formed from a magnesium alloy of magnesium and 6.25 % by weight aluminium, which was produced using the system according to the invention at 275°C and with use of a tensile force of 50N,
- Fig. 4: a comparison of the dilatability (RFD in % above the specification) of implant scaffolds which
a) were produced from extruded tubular semi-finished products from a magnesium alloy, wherein the semi-finished products were produced without the introduction according to the invention of a tensile stress and/or torsional stress, or which
b) were produced from extruded tubular semifinished products from a magnesium alloy, wherein the semi-finished products were produced using the system according to the invention.

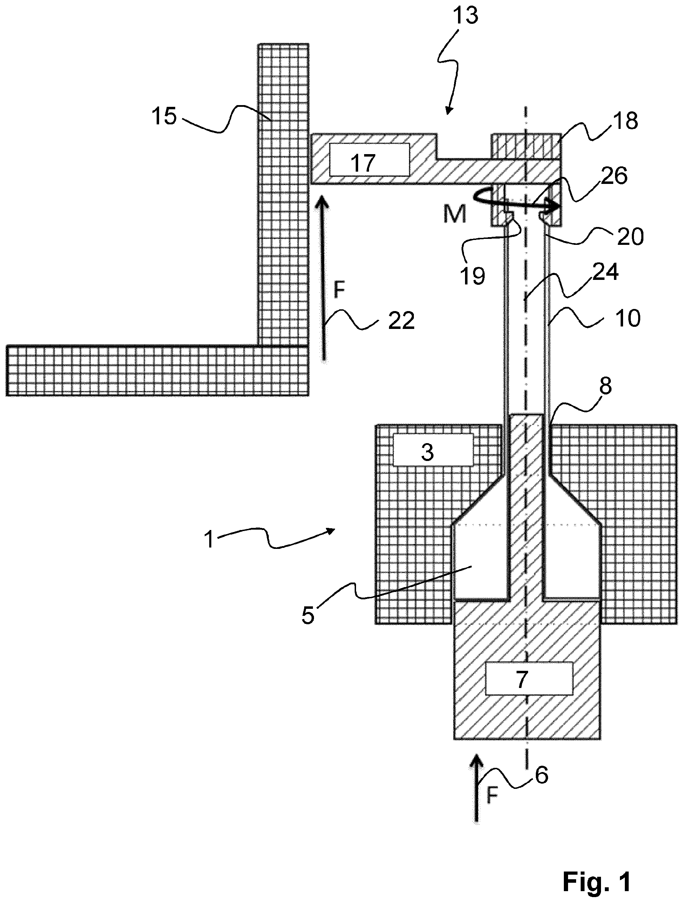

The system according to the invention shown in Fig. 1 is intended for producing a tubular semi-finished product for a scaffold of an implant. The system has an extrusion device 1 with a heated die 3. The material 5 to be extruded is arranged in a recess of the die. The material 5 is pressed from a shaping outlet opening 8 using a ram 7. The outlet opening 8, which is created from the interaction of the ram 7 and the die 3, has a hollow-cylindrical form, so that a tubular or hollow-cylindrical semi-finished product 10 is created. The die 3 heats the material 5, for example in a temperature range between 240°C and 450°C.

Fig. 1 also shows a tube drawing device 13 with an L-shaped holder 15 and a slide (puller) 17 guided in a rail of the holder 15. A rotary head 18 with a pneumatic drive (not shown) is arranged on an arm protruding from the slide 17, which drive is connected to a clamping device 19 so that the slide 17 is also connected to the clamping device 19. At its end opposite the die 3 of the extrusion device 1, the semi-finished product 10 that has exited from the outlet opening 8 is engaged by the clamping device 19, which is fixedly connected to the semi-finished product 10 in this portion 20.

The slide 17 is moved upwardly (see arrow 22) in the view of Fig. 1 in the rail of the holder 15, for example by means of a pneumatic drive. The slide 17 generates a tensile force F (see arrow 22), which is transferred via the clamping device 19 to the semi-finished product 10 and brings about a tensile stress in the material of the semi-finished product 10. The pneumatic drive for the slide 17 can comprise electronic proportional valves, by means of which a force/distance diagram can be predefined and implemented.

Alternatively or additionally, the clamping device 19 held rotatably in the arm of the slide 17 is rotated by means of the rotary head 18 by a further pneumatic drive (not shown) about a longitudinal axis which runs parallel to the longitudinal axis 24 of the tubular semi-finished product 10 (for example coincides therewith). This is shown in Fig. 1 by the arrow 26. A torsion moment M is hereby generated, which is transferred via the clamping device 19 to the semi-finished product 10 and brings about a torsional stress in the material of the semi-finished product 10. The torsion moment is adjusted as a function of the length of the semi-finished product 10.

It should be emphasised at this juncture that, by means of the tube drawing device 13 according to the invention acting on the semi-finished product 10, a tensile stress or a torsional stress or both stresses can be generated in the material of the semi-finished product 10 exiting from the extrusion device 1. The tensile force or the torsion moment act here directly on the semi-finished product exiting from the extrusion device.

The clamping device 19, in the starting state, is arranged on the semi-finished product at a distance from the outlet opening 8, wherein the distance is at least 0.2 mm.

Alternatively, an extruded and initially cooled tubular semi-finished product can be tempered by means of a heating device in a temperature range between 180°C and 270°C and a tensile stress and/or torsional stress is then introduced into the heated material of the semi-finished product by means of the above-described pipe drawing device 13.

The tensile stress introduced into the material of the semi-finished product 10 lies in the range of from 20 N/mm² to 100 N/mm², preferably 40 N/mm², and the introduced torsional stress lies in the range of 0-100% of the introduced tensile stress, preferably 50% of the tensile stress.

The semi-finished product 10 provided with a tensile stress and/or a torsional stress is then cooled in both embodiments.

The implant according to the invention, for example a stent, can be produced from the semi-finished product 10 in the known manner by means of laser cutting and subsequent electropolishing.

The material of the semi-finished product 10 and thus of the scaffold or the implant is in this embodiment a magnesium alloy, for example WE43, magnesium-zinc-aluminium, magnesium-aluminium or magnesium-zinc-calcium.

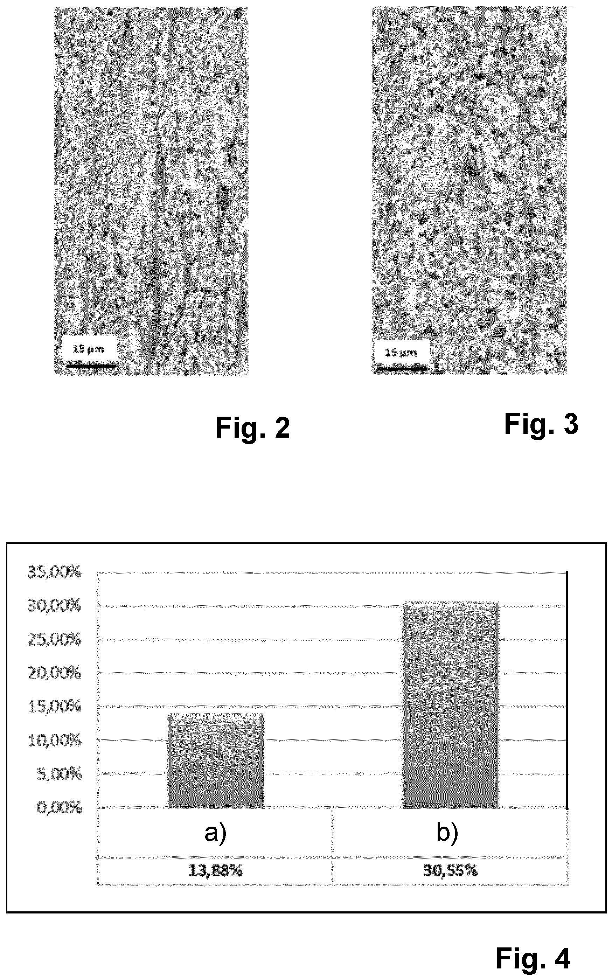

As has already been explained above, the introduced stresses cause a grain refinement, with the adjacent grains of the microstructure having a slightly different orientation. The grains are therefore no longer - as in the conventional methods - oriented only in the preferred direction along the longitudinal axis of the tubular semi-finished product (see Fig. 2), but are smaller and have a mixed orientation. This can be seen very well in Fig. 3, which shows the microstructure once tensile and/or torsional stresses have been introduced by means of the tube drawing device 13 according to the invention. More sliding planes are thus available - in the event of plastic deformation, stress peaks in a grain are removed again by the next grain. In addition, the speed of growth of cracks reduces as a result of the torsion moments contained in the material, since cracks in the grains are forced to change their direction. The introduced tensile and/or torsional stresses additionally ensure an increased recrystallisation rate (dynamic recrystallisation). Due to the stresses, the nucleation during the forming is facilitated. A greater number of new grains is thus created, whereby the proportion of the recrystallised microstructure increases (see Fig. 3). As a result of this process, isotropic material properties are promoted without grain growth as a result of thermomechanical process influencing. In addition, the microstructure homogeneity in the overall tubular semi-finished product 10 is increased, such that a uniform texture is produced along the entire tube length and the entire tube cross section also in the circumferential direction. This in turn increased the isotropic response of a scaffold produced from the semi-finished product during the crimping or the dilation as a prerequisite for use of the scaffold as an implant in the treated bodily lumen.

The improvement of the material properties is evident from the graph shown in Fig. 4. By measuring what is known as the "rated fracture diameter (RFD)", i.e. the diameter of the scaffold obtainable with statistic certainty during dilation until breakage, it is possible to assess the dilatability. Fig. 4 shows a comparison of the RFD for scaffolds produced from conventional semi-finished products (a)) and scaffolds whose semi-finished product was produced by means of the method according to the invention (b)). An increase of the dilatability by more than 16% is determined.

### List of reference signs

- 1: extrusion device
- 3: heated die
- 5: material to be extruded
- 7: ram
- 8: outlet opening
- 10: tubular semi-finished product
- 13: tube drawing device
- 15: holder
- 17: slide (puller)
- 18: rotary head
- 19: clamping device
- 20: portion of the semi-finished product 10
- 22: arrow
- 24: longitudinal axis of the semi-finished product 10
- 26: arrow

## Claims

1. A method for producing a tubular semi-finished product (10) for a scaffold of an implant, wherein the semi-finished product (10) consists of a magnesium alloy, said method having the following steps:
a) extruding the tubular semi-finished product (10) by means of a heated die (3),
b) introducing into the material of the semi-finished product (10) exiting from the die (3) a tensile stress by means of a tube drawing device (13), which has a clamping device (19), wherein the clamping device is fixed on a predefined portion (20) of the tubular semi-finished product (10) and the tensile force generated by the tube drawing device (13) transfers to the semi-finished product (10), **characterised in that** the temperature applied to the material of the semi-finished product heated in the die (3) is between 240 °C and 290 °C.

2. The method according to claim 1, **characterised in that** tensile stress introduced into the material of the semi-finished product (10) lies in the range of from 20 N/mm² to 100 N/mm², preferably 40 N/mm².

3. The method according to one of the preceding claims, **characterised in that** the tensile stress is generated by means of a slide (17) of the tube drawing device (13), which slide is movable on a guide, wherein the clamping device (19) is connected to the slide (17) and the slide (17) is driven pneumatically.

4. A system comprising a device (13) for producing a tubular semi-finished product (10) for a scaffold of an implant for use with a heated die (3) for extruding a tubular semi-finished product (10), wherein the device (13) has a clamping device (19) and is designed in such a way that it generates a tensile stress in the material of the semi-finished product (10), wherein the tensile force generated for this purpose by the device (13) is transferable by means of the clamping device (19) to the material of the semi-finished product (10) exiting from the die (3), wherein the clamping device (19) is fixable on a predefined portion (20) of the tubular semi-finished product (10) and
an extrusion device (1) with a heated die (3) for extruding the tubular semi-finished product (10), **characterised in that** the device (13) has a slide (17) which is movable on or in a guide, which generates the tensile force, and which is connected to the clamping device (19) and the slide (17) is driven pneumatically..

## Patentansprüche

1. Verfahren zur Herstellung eines rohrförmigen Halbzeugs (10) für ein Implantatgerüst, wobei das Halbzeug (10) aus einer Magnesiumlegierung besteht, mit folgenden Schritten:
a) Strangpressen des rohrförmigen Halbzeugs (10) mittels einer beheizten Matrize (3),
b) Einbringen einer Zugspannung in das aus der Matrize (3) austretende Material des Halbzeugs (10) mittels einer Rohrziehvorrichtung (13), die eine Klemmvorrichtung (19) aufweist, wobei die Klemmvorrichtung an einem vorgegebenen Abschnitt (20) des rohrförmigen Halbzeugs (10) befestigt ist und die von der Rohrziehvorrichtung (13) erzeugte Zugkraft auf das Halbzeug (10) übertragen wird, **dadurch gekennzeichnet, dass** die auf das Material des in der Matrize (3) erhitzten Halbzeugs angewandte Temperatur zwischen 240 °C und 290 °C liegt.

2. Verfahren nach Anspruch 1, **dadurch gekennzeichnet, dass** Zugspannung, die in das Material des Halbzeugs (10) eingebracht wird, im Bereich von 20 N/mm² bis 100 N/mm², vorzugsweise 40 N/mm², liegt.

3. Verfahren nach einem der vorstehenden Ansprüche, **dadurch gekennzeichnet, dass** die Zugspannung mittels eines Schlittens (17) der Rohrziehvorrichtung (13) erzeugt wird, wobei der Schlitten auf einer Führung bewegbar ist, wobei die Klemmvorrichtung (19) mit dem Schlitten (17) verbunden ist und der Schlitten (17) pneumatisch angetrieben wird.

4. System mit einer Vorrichtung (13) zur Herstellung eines rohrförmigen Halbzeugs (10) für ein Gerüst eines Implantats zur Verwendung mit einer beheizten Matritze (3) zum Extrudieren eines rohrförmigen Halbzeugs (10), wobei die Vorrichtung (13) eine Klemmvorrichtung (19) aufweist und derart ausgebildet ist, dass sie eine Zugspannung im Material des Halbzeugs (10) erzeugt, wobei die hierzu von der Vorrichtung (13) erzeugte Zugkraft mittels der Klemmvorrichtung (19) auf das aus der Matrize (3) austretende Material des Halbzeugs (10) übertragbar ist, wobei die Klemmvorrichtung (19) an einem vorgegebenen Abschnitt (20) des schlauchförmigen Halbzeugs (10) und eine Extrusionsvorrichtung (1) mit einer beheizten Matritze (3) zum Extrudieren des rohrförmigen Halbzeugs (10) fixierbar ist, **dadurch gekennzeichnet, dass** die Vorrichtung (13) einen Schlitten (17) aufweist, der auf oder in einer Führung bewegbar ist, der die Zugkraft erzeugt, und der mit der Klemmvorrichtung (19) verbunden ist und der Schlitten (17) pneumatisch angetrieben ist.

## Revendications

1. Procédé de production d'un produit semi-fini (10) tubulaire pour un échafaudage d'une prothèse, dans lequel le produit semi-fini (10) est constitué d'un alliage de magnésium, ledit procédé ayant les étapes suivantes consistant à :
a) extruder le produit semi-fini (10) tubulaire au moyen d'une filière chauffée (3),
b) introduire dans le matériau du produit semi-fini (10) sortant de la filière (3) une contrainte de traction au moyen d'un dispositif d'étirement de tube (13), qui a un dispositif de serrage (19), dans lequel le dispositif de serrage est fixé sur une partie prédéfinie (20) du produit semi-fini (10) tubulaire et la force de traction générée par le dispositif d'étirement de tube (13) transfère le produit semi-fini (10), **caractérisé en ce que** la température appliquée au matériau du produit semi-fini chauffé dans la filière (3) est entre 240 °C et 290 °C.

2. Procédé selon la revendication 1, **caractérisé en ce que** la contrainte de traction introduite dans le matériau du produit semi-fini (10) est comprise dans la plage allant de 20 N/mm² à 100 N/mm², de préférence de 40 N/mm².

3. Procédé selon l'une des revendications précédentes, **caractérisé en ce que** la contrainte de traction est générée au moyen d'une coulisse (17) du dispositif d'étirement de tube (13), laquelle coulisse peut être déplacée sur un guide, dans lequel le dispositif de serrage (19) est relié à la coulisse (17) et la coulisse (17) est entraînée de manière pneumatique.

4. Système comprenant un dispositif (13) destiné à produire un produit semi-fini (10) tubulaire pour un échafaudage d'une prothèse destiné à une utilisation avec une filière chauffée (3) afin d'extraire un produit semi-fini (10) tubulaire, dans lequel le dispositif (13) a un dispositif de serrage (19) et est conçu de telle sorte qu'il génère une contrainte de traction dans le matériau du produit semi-fini (10), dans lequel la force de traction générée à cette fin par le dispositif (13) peut être transférée au moyen du dispositif de serrage (19) au matériau du produit semi-fini (10) sortant de la filière (3), dans lequel le dispositif de serrage (19) peut être fixé sur une partie prédéfinie (20) du produit semi-fini (10) tubulaire et
un dispositif d'extrusion (1) avec une filière chauffée (3) destiné à extruder le produit semi-fini (10) tubulaire, **caractérisé en ce que** le dispositif (13) a une coulisse (17) qui peut être déplacée sur ou dans un guide, qui génère la force de traction, et qui est reliée au dispositif de serrage (19) et la coulisse (17) est entraînée de manière pneumatique.
